# EUROPEAN PATENT APPLICATION

(11) **EP 0 585 509 A1**
(43) Date of publication of application: **09.03.1994**
(21) Application number: 92830671.1
(22) Date of filing: 14.12.1992
(51) Int. Cl.: A61K 7/26, A61K 35/78, A61K 35/00

(54) **Sanadent**

(71) Applicant: Pennisi, Mario, Paterno, Catania (IT)
(72) Inventor: Pennisi, Mario, Paterno, Catania (IT)

(57) **Abstract**

SANADENT is composed by natural estracts concerning problems only with the teeths.

SANADENT is useful for: teethpain, to end the pain, abscesses, inflammated gums, for diabetics who can't take chemical medecines, for pregnant women who can't take chemical medecines.

SO FOR THESE CASES THESE PRODUCTS ARE EXCELLENT.

## Description

SANADENT is composed by natural estracts concerning problems only with the teeths.

SANADENT is useful for : teethpain, to end the pain, abscesses; inflammated gums, for diabetics who can't take chemical medicines, for pregnant women who can't take chemical medicines.

SO FOR THESE CASES THESE PRODUCTS ARE EXCELLENT.

### 2) THE FEATURES

The patient can use it very well at home, in a very simple way like prevention or resolving a problem that might present itself.

### 3) USING MODALITIES

After every meal, in the morning at lunch or at dinner, take a part of SANADENT, about 125 gr in a little pot and heat it up. It's important that SANADENT is warm, then pour it out in a glass and then take a sip and make some gargles with it. To repeat several times. After, the patient must limit himself with the food and the drinking for at least four hours. Only water is admitted. The treatment will last for three days. Once startting the cure the patient musn't brush his teehs and smoke.

At the end of the treatment the patient returns to normallity and can start eating, drinking and brush his teeths.

SANADENT will be consumed in four meals.

In case of no results, (the problems exist from patient to patient) then: the person in question cant't assimilate or assimilates it lesser.

## Claims

1. SANADENT is composed by natural estracts, salt, water, extracts of vinegar and extracts of hazelnuts, concerning problems only with the teeths.
